# EUROPEAN PATENT APPLICATION

(11) **EP 1 754 512 A2**
(43) Date of publication of application: **21.02.2007**
(21) Application number: 06117344.9
(22) Date of filing: 17.07.2006
(51) Int. Cl.: A61N 1/40, A61N 1/34

(54) **Method and apparatus for diagnosing and treating neural dysfunction**

(30) Priority: 18.08.2005 US 709235 P
(71) Applicant: Neurotherm, Inc., Middleton, MA 01949 (US)
(72) Inventor: Rittman, William J., III, Lynnfield, MA 01940 (US)
(74) Representative: Kihn, Pierre Emile Joseph

(57) **Abstract**

A method and apparatus for diagnosing and treating neural dysfunction is disclosed, which comprises taking the energy output from a high frequency generator module and delivering this energy as in a pulsed manner to a treatment electrode. In one exemplary embodiment, a temperature set point is utilized, and the pulses are modified to limit the energy delivered such that the temperature is limited. One exemplary method of modifiying pulses includes reducing the amplitude of the pulses. Another exemplary method of modifying pulses includes reducing pulse width. Another exemplary embodiment of modifying pulses includes only delivering full width and amplitude pulses.

## Description

### FIELD

The presently described system relates generally to the advancement of medical technology, processes, and systems for the treatment of pain, neurological disorders, and other clinical maladies related to neural dysfunction. More specifically, the present disclosure is directed at a system for producing therapeutic lesions or tissue alterations by means of a high frequency generator connected to a patient. In below-described exemplary embodiments, therapeutic energy is delivered in a pulsed rather than continuous manner. Various specific exemplary embodiments of this device accommodate specific exemplary clinical applications and designs.

### BACKGROUND

The general use of radiofrequency and high frequency generator systems which deliver electrical output to electrodes that are connected to a patient's body is known in the clinical literature and art.

By reference, an example of radiofrequency heat lesioning generators used in clinical practice for the treatment of neural disorders is the Radionics RFG-3C+ (Burlington MA).

This device is capable of delivering high frequency energy to patient tissue via an adapted electrode, and associated ground or reference electrode. This device is also capable of delivering low frequency stimulation pulses that are used to accurately localize the electrode placement before treatment.

Parameters that may be measured by these devices include impedance. HF voltage, HF current, HF power, and electrode tip temperature. Parameters that may be set by the user include time of energy delivery, desired electrode temperature, stimulation frequencies and durations, and level of stimulation output. In general, electrode temperature is a parameter that may be controlled by the regulation of high frequency output power.

These devices have various user interfaces that allow the selection of one or more of these treatment parameters, as well as various methods to display the parameters mentioned above.

In a one application of these devices, a patient complains of back pain, or some other pain of nocioceptive or neuropathic origin. A doctor then performs diagnostic blocks with local anesthetic by injecting the anesthetic into the areas that is suspected of generating the pain. If the patient receives temporary pain relief from these injections the doctor concludes that the pain generators were in the location where he made these injections. Unfortunately, the origin of pain is poorly understood; perceived pain at a certain level in the back, for instance, can actually be created from many different and multiple sources.

Once a location has been identified, the clinician will decide to deliver high frequency energy to this location to permanently destroy the pain generator. A ground or reference plate will be placed on the patient's thigh to provide a return path for the high frequency energy. An insulated electrode with a small un-insulated tip will be placed at the expected target. Stimulation pulses will be delivered at a sensory frequency (typically 50 Hz), and a stimulation voltage will be placed on the electrode. The clinician is looking for a very low threshold of response from the patient (e.g., less than .5 V) to ensure that the electrode is close to the sensory nerves. They will then perform a stimulation test at a muscle motor frequency (e.g., 2 Hz), and increase the stimulation voltage output to 2v. In this instance, they are looking for no motor response in the patient's extremities as this would indicate the electrode was too close to the motor nerves. Treatment in this area could cause paralysis. Upon successful completion of these tests, high frequency energy is typically delivered for one or more minutes, while maintaining an electrode tip temperature between 70 and 90 degrees. Alternatively, high frequency energy may be delivered for one or more minutes, but in a pulsed mode where the high frequency energy is on for a short period of time and off for a long period of time, thus not producing any appreciable heating (reference is made to commonly assigned U.S. Patent. No. 6,161,048, the entire contents of which are specifically incorporated by reference herein).

### SUMMARY

The above-described and other disadvantages of the art are overcome and alleviated by the present method and system for taking the energy output from a high frequency generator module and delivering this energy as in a pulsed manner to a treatment electrode. In one exemplary embodiment, a temperature set point is utilized, and the pulses are modified to limit the energy delivered such that the temperature is limited. One exemplary method of modifying pulses includes reducing the amplitude of the pulses. Another exemplary method of modifying pulses includes reducing pulse width. Another exemplary embodiment of modifying pulses includes only delivering full width and amplitude pulses. These exemplary embodiments will be more fully described hereinbelow.

The above discussed and other features and advantages of the present system will be appreciated and understood by those skilled in the art from the following detailed description and drawings.

### BRIEF DESCRIPTION OF THE DRAWINGS

Referring now to the figures, which are exemplary embodiments and wherein the like elements are numbered alike:

Figure 1 represents a simple exemplary embodiment of the presently described system;

Figure 2 illustrates an exemplary temperature feedback control mechanism;

Figure 3 is another exemplary embodiment showing the representation of the temperature of an electrode in graphical form;

Figure 4 is another exemplary embodiment which also illustrates the graphing of the EMG signal;

Figure 5 is another exemplary embodiment showing one method of representing pre and post-treatment sensory stimulation thresholds; and

Figure 6 is another exemplary embodiment showing three distinct mode selections, as well as an exemplary method to record sensory stimulation thresholds.

### DETAILED DESCRIPTION

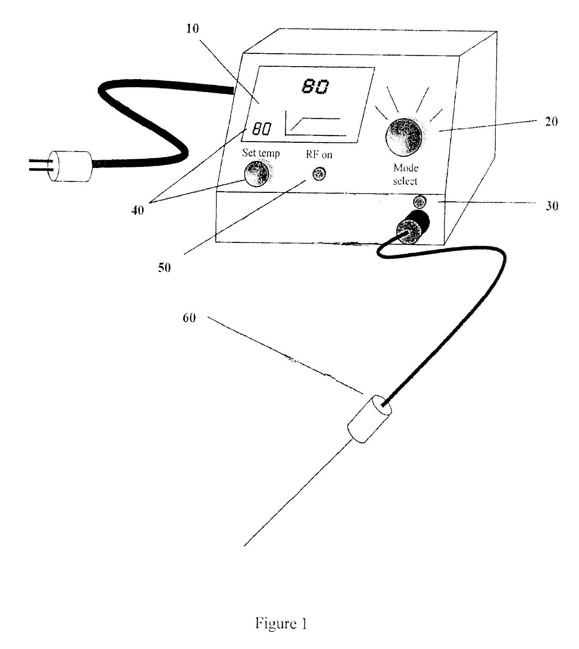

Referring to Figure 1, an exemplary embodiment is illustrated. Mode select switch 20 allows the user to selectively connect an electrode 60, to a high frequency power source. This permits the high frequency power source to selectively be connected to the electrode for the purpose of doing impedance measurements or stimulation threshold testing. The high frequency energy is delivered to the electrode and the electrode temperature is measured and compared to the user set temperature, represented by 40 in Figure 1. In this embodiment the electrode temperature is displayed on a two-dimensional graphics panel identified by 10 in the figure. Also within the graphics display is a representation of temperature vs. time displayed in graphic format. An indicator light, represented by 30 in Figure 1, indicates whether the electrode is active at that particular moment.

It is very important to note two things from this figure -- one is that to the high frequency power source that delivers the high frequency energy and/or low frequency stimulation pulses could be incorporated into this device or could be a separate stand-alone unit, with this device interposed between the high frequency power source and the electrodes. Though the figure shows this device as being AC line connected (that is requiring an electrical outlet for the unit to be plugged into), a battery-operated device would also contemplated.

It should also be understood that mode selection could be done in many ways and the features of this user interface could be achieved with or without displays, and could use up/ down pushbuttons rather than rotatable selector knobs. For instance, mode select could connect the electrode to the high frequency device, and could also have a position may connect the electrode to an EMG measuring circuit, where the EMG signal may be displayed on a two-dimensional graphics display. An additional position on the mode select would be high frequency energy delivery where either continuous or pulsed high frequency energy may be delivered to the electrode, and a feedback circuit may be incorporated to maintain the electrode tip at a temperature equal to set temp.

The present disclosure recognizes that where pulsed high frequency is delivered to an electrode, and where a temperature set point is utilized, temperature regulation at the electrode is problematic. The present disclosure recognizes that each pulse delivered should be the same amplitude and pulse width. Three exemplary methods of limiting the energy delivered (and thus, regulating the temperature) are described herein.

One exemplary method of limting the energy delivered comprises reducing the amplitude of the pulses. Another exemplary method comprises reducing the pulse width of the pulses. The above methods may be effective to limit the energy delivered even if, as often occurs, the amplitude of the pulses or the pulse shapes vary during treatment and among different patients.

Another exemplary method comprises delivering only substantially full width and amplitude pulses. In an exemplary implementation of this method, if a temparature set point is reached, no pulses are delivered until the temperature falls below the set point. This is a very uniform method of controlling delivery of pulses. Using this technique, however, results in delivering varying numbers of pulses for a defined treatment time. This method may therefore be further refined by using a treatment scheme wherein pulses are counted (i.e., counting pulses or "doses") as opposed to defining a time of treatment. In such scheme, treatment is not measured in seconds, but rather in pulses, e.g., 240 pulses or 300 pulses. Using such techique, temperature may be regulated and uniform delivery of treatment is attained.

With further regard to the instrument illustrated at Figure I, it should also be noted there are many ergonomic manifestations of this invention and it would be possible to add additional displays, buttons, and/or indicators to allow and/or assist the operator in controlling the device. For instance, Figure 1 has an RF on indicator light, represented by 50, which will indicate whenever high frequency energy is being delivered to the electrode output.

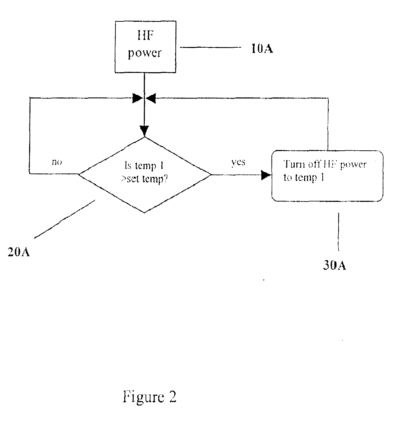

Figure 2 is an exempalry logic control diagram indicating a basic exemplary feedback mechanism for the temperature control electrode. HF power, identified as 10A in the figure, is delivered system. The temperature of the electrode receiving this HF energy, as well as the user set temperature, is measured and a decision point is reached, represented by 20A in the figure. If the electrode temperature is greater than the user set temperature, the HF power is turned off to the electrode. This action is represented by block 30A in Figure 2. Then this process starts all over again, where the electrode temperature is once again compared to the user set temperature. Conversely, if the measured temperature for that particular electrode is less than the user set temperature the HF remains on, and again, the electrode temperature is subsequently compared to the user set temperature. In this way temperature feedback is realized, which will maintain the electrode temperature at the same level as the user set temperature.

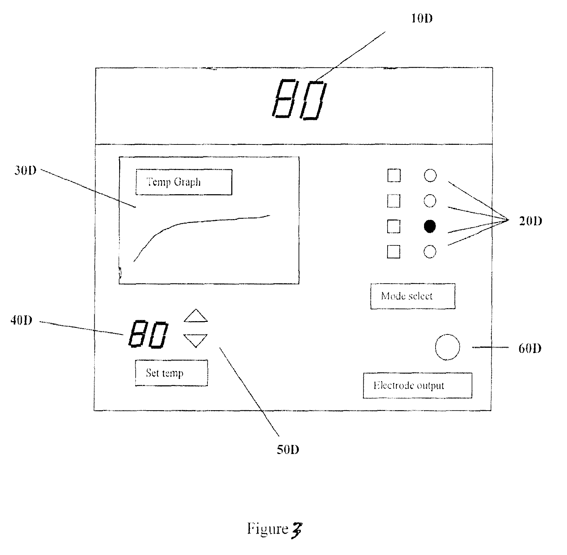

In Figure 3, another exemplary embodiment of the user interface is illustrated. As identified by 10D and 40D, it is clear that electrode temperature and/or other pertinent parameters need not be displayed on a two-dimensional screen. These could be represented, for instance, by LED or LCD digits. 30D again represents a two-dimensional graphics display, in this case displaying temperature. Again, a graphics display is not necessary to realize the presently described system and method. To demonstrate exemplary options for user interface, the mode selector has been represented by a series of buttons that are associated with indicator lights identified as 20D in the figure and Set temp has been identified as up/ down arrows as shown by 50D. The electrode output has been schematically represented by 60D.

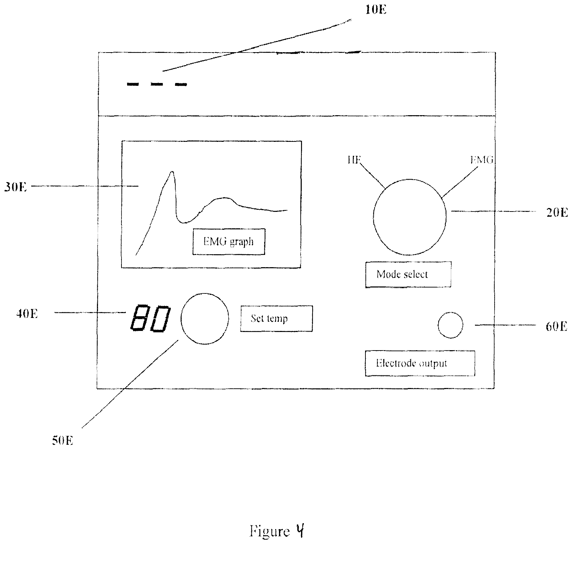

In figure 4, additional exemplary embodiments of the device are shown where, this time, the mode select 20E, has a position for EMG in addition to a High Frequency energy delivery position. On the two-dimensional display, an EMG signal can be represented, thus identifying electrophysiological activity of a nerve before and/or after the High Frequency treatment. For completeness, 60E identifies the electrode output, were once again three have been illustrated, although any number greater than 1 is possible with the present sytem and method. The Set temp user interface has been represented in this diagram as a knob 50E, though as mentioned earlier there are other comtemplated ways to achieve this user interface. 40E identifies the actual set temperature. 10E is indicating that the temperature displays of the electrodes ( -- ) is not relevant since they would indicate body temperature (37 ° C), though this temperature could be displayed if desired.

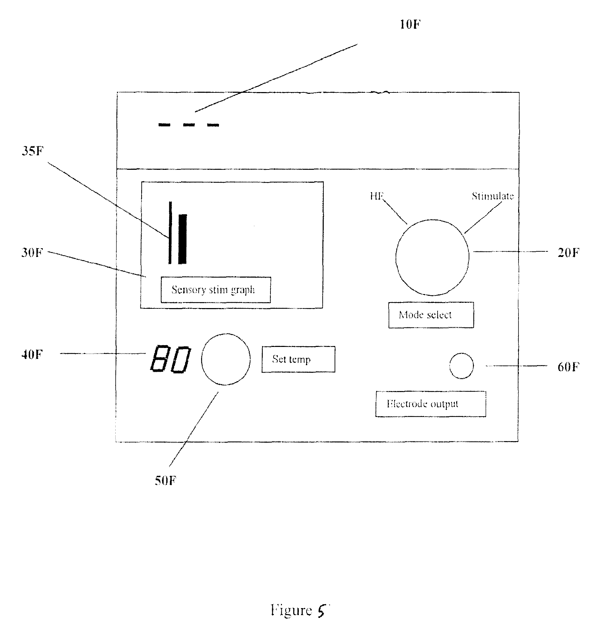

Figure 5 is an exemplary embodiment showing a sensory stimulation graph 30F, being displayed on the device. In this particular diagram, the electrode has associated with it a thin line and a fat line 35F indicating pre- and post- stimulation sensory thresholds for the electrode. Again, there are many contemplated ways that these parameters could be represented, and this is just an example of one or many ways in which to achieve a representation of these parameters that are identifiable to the user. The mode select switch, identified as 20F, has settings for both High Frequency energy and stimulation. The dashes (--), indicated by 10F in the figure, represent temperature, which is irrelevant in this mode, since with no energy delivery there is no therapeutic heating and the electrode will be reading body temperature (which could of course be displayed). The electrode output, represented by 60F, once again indicate an electrode connection. Set temp is represented by 5F in the figure, and its associated value is represented by 40F in the figure and is depicted as a two digit display.

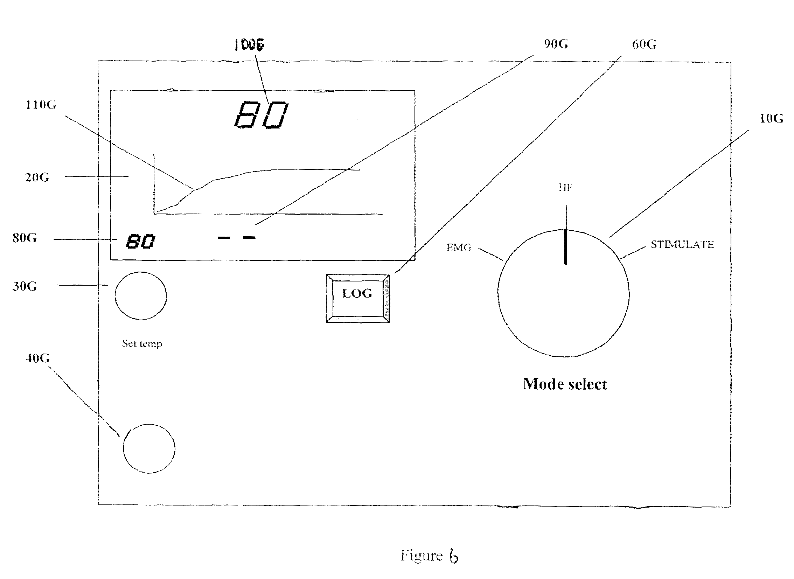

Figure 6 is another exemplary embodiment. Illustrated is a mode select button, 10G, winch allows the user to select between EMG, HE, and stimulate modes. When stimulate or EMG mode is selected, a digit(s) represented by 90G, indicates whether the electrode is selected. In this embodiment, the user set temperature is identified as a knob indicated by 30G, and the set temperature value is represented by 80G in the figure, and is incorporated within a two-dimensional graphics display 20G. A time vs. temperature graph is indicated by 110G in the figure, and the electrode temperature, if HF is selected on the mode select, is indicated by 100G in the figure. 40G once again indicates an electrode output. 60G identifies a log button. This button is used in stimulate mode, since the user must identify what stimulation voltage threshold is to be saved for future display.

While the disclosure has been described with reference to exemplary embodiments, it will be understood by those skilled in the art that various changes may be made and equivalents may be substituted for elements thereof without departing from the scope of the disclosure. In addition, many modifications may be made to adapt a particular situation or material to the teachings of the disclosure without departing from the essential scope thereof. Therefore, it is intended that the disclosure not be limited to the particular embodiment disclosed as the best mode contemplated for carrying out this disclosure, but that the disclosure will include all embodiments falling within the scope of the appended claims.

## Claims

1. A method for performing nerve modification procedures on a patient's body, comprising:
providing a device adapted for connection to an electrode; and
supplying a high frequency energy source to said at least one electrode in a pulsed manner, wherein said pulsed high frequency energy is modified to limit the energy delivered to said electrode.

2. A method in accordance with claim 1, wherein said high freqency energy is modified by reducing the amplitude of pulses.

3. A method in accordance with claim 1, wherein said high frequency energy is modified by reducing the pulse width of pulses.

4. A method in accordance with claim 1, wherein said high frequency energy is modified by delivering substantially only full width and amplitude pulses.

5. A method in accordance with claim 4, further comprising providing a user-defined temperature set point, wherein said set point is utilized by said device as a guide for delivery of energy.

6. A method in accordance with claim 5, further comprising a temperature sensor provided in a tip portion of said electrode, and wherein said device docs not deliver pulses unless said temperature in said electrode tip portion falls below said set point.

7. A method in accordance with claim 1, wherein a treatment log is recorded or documented according to the number of pulses delivered by said electrode.

8. A method in accordance with claim 1, further comprising a temperature sensor provided in a tip portion of said electrode and a user-defined temperature set point provided by said device, and wherein said device does not deliver pulses unless said temperature in said electrode tip portion falls below said set point.

9. A method in accordance with claim 8, further comprising a feedback control circuit configured to regulate energy delivery to said electrode so as to maintain a user settable temperature at the electrode tip portion.

10. A device for performing nerve modification procedures on a patient's body, comprising:
a device adapted for connection to an electrode; and
a high frequency energy source operatively associated with said device, wherein said high frequency energy source or said device is configured to provide high frequency energy to said at least one electrode in a pulsed manner, and wherein said pulsed high frequency energy is modified to limit the energy delivered to said electrode.

11. A device in accordance with claim 10, wherein said high freqency energy source or said device is configured to modify said pulsed high energy source by reducing the amplitude of pulses.

12. A device in accordance with claim 10, wherein said high freqency energy source or said device is configured to modify said pulsed high energy source by reducing the pulse width of pulses.

13. A device in accordance with claim 10, wherein said high fregency energy source or said device is configured to modify said pulsed high energy source by delivering substantially only full width and amplitude pulses.

14. A device in accordance with claim 13, further comprising a user-defined temperature set point on said device, wherein said set point is utilized by said device as a guide for delivery of energy.

15. A device in accordance with claim 14, further comprising a temperature sensor provided in a tip portion of said electrode, and wherein said device does not deliver pulses unless said temperature in said electrode tip portion falls below said set point.

16. A device in accordance with claim 10, wherein a treatment log is recorded or documented by said device according to the number of pulses delivered by said electrode.

17. A device in accordance with claim 10, further comprising a temperature sensor provided in a tip portion of said electrode and a user-defined temperature set point on said device, and wherein said device does not deliver pulses unless said temperature in said electrode tip portion falls below said set point.

18. A device in accordance with claim 10, further comprising a feedback control circuit configured to regulate energy delivery to said electrode so as to maintain a user settable temperature at the electrode tip portion.

19. A device in accordance with claim 10, wherein said electrode is at least partially electrically insulated over at least part of a shaft of said electrode.

20. A device in accordance with claim 10, further comprising a user interface which allows the user to set to the desired temperature of said electrode.

21. A device in accordance with claim 10, wherein the said high frequency energy source is also capable of delivering low frequency (1-1000 Hz) stimulation pulses.

22. A device in accordance with claim 20, wherein the said user interface further comprises at least one display configured to display graphic representation of the temperature of said electrode tip, and/or time versus temperature of each of said electrode.

23. A device in accordance with claim 22, wherein said user interface can also display EMG signals that are recorded from said electrode.

24. A device in accordance with claim 22, wherein the user interface can also deliver audible representations of the EMG signal.

25. A device in accordance with claim 22, wherein the user interface can also record and display user selectable sensory stimulation thresholds from said electrode.
